# EUROPEAN PATENT SPECIFICATION

(11) **EP 2 851 074 B1**
(45) Date of publication and mention of the grant of the patent: **16.08.2017**
(21) Application number: 13790165.8
(22) Date of filing: 06.05.2013
(51) Int. Cl.: A61K 31/485, A61K 31/195, A61K 31/167, A61K 31/197, A61K 31/616, A61P 29/00

(54) **ANALGESIC PHARMACEUTICAL COMPOSITION**
ANALGETISCHE PHARMAZEUTISCHE ZUSAMMENSETZUNG
COMPOSITION PHARMACEUTIQUE ANALGÉSIQUE

(30) Priority: 17.05.2012 CN 201210154087
(43) Date of publication of application: 25.03.2015
(73) Proprietor: Gao, Tongqiang, Haidian Distrct, Beijing 100098 (CN); Xu, Xiaojun, 14135 Huddinge (SE); Hao, Jingxia, 14135 Huddinge (SE); Gao, Tianle, 17168 Solna (SE); Wiesenfeld-Hallin, Zsuzsanna, 11221 Stockholm (SE)
(72) Inventor: Gao, Tongqiang, Haidian Distrct, Beijing 100098 (CN); Xu, Xiaojun, 14135 Huddinge (SE); Hao, Jingxia, 14135 Huddinge (SE); Gao, Tianle, 17168 Solna (SE); Wiesenfeld-Hallin, Zsuzsanna, 11221 Stockholm (SE)
(74) Representative: Groth & Co. KB
(86) International application number: PCT/CN2013/075205
(87) International publication number: WO 2013/170711

(56) References cited:
- CN-A- 1 450 883
- CN-A- 1 673 212
- CN-A- 101 066 949
- CN-A- 101 352 412
- CN-A- 101 461 821
- CN-A- 102 813 924
- DATABASE WPI Week 200828 Thomson Scientific, London, GB; AN 2008-D85614 XP002739075, & CN 101 066 949 A (UNIV GUANGDONG IND) 7 November 2007 (2007-11-07)
- XU MIN ET AL: "Sinomenine Versus NSAIDs for the Treatment of Rheumatoid Arthritis: A Systematic Review and Meta-Analysis", PLANTA MEDICA, vol. 74, no. 12, 5 August 2008 (2008-08-05), pages 1423-1429, XP055186307, ISSN: 0032-0943, DOI: 10.1055/s-2008-1081346
- ZHOU H ET AL: "Sinomenine ameliorates arthritis via MMPs, TIMPs, and cytokines in rats", BIOCHEMICAL AND BIOPHYSICAL RESEARCH COMMUNICATIONS, ACADEMIC PRESS INC. ORLANDO, FL, US, vol. 376, no. 2, 14 November 2008 (2008-11-14), pages 352-357, XP025495738, ISSN: 0006-291X, DOI: 10.1016/J.BBRC.2008.08.153 [retrieved on 2008-09-07]
- WANG QUANXING ET AL: "Immunosuppressive and anti-inflammatory activities of sinomenine", INTERNATIONAL IMMUNOPHARMACOLOGY, ELSEVIER, AMSTERDAM, NL, vol. 11, no. 3, 23 November 2010 (2010-11-23), pages 373-376, XP028152240, ISSN: 1567-5769, DOI: 10.1016/J.INTIMP.2010.11.018 [retrieved on 2010-11-22]
- ATTAL N ET AL: "Chronic neuropathic pain management in spinal cord injury patients. What is the efficacy of pharmacological treatments with a general mode of administration? (oral, transdermal, intravenous)", ANNALS OF PHYSICAL AND REHABILITATION MEDICINE, ELSEVIER, AMSTERDAM, NL, vol. 52, no. 2, March 2009 (2009-03), pages 124-141, XP026022405, ISSN: 1877-0657, DOI: 10.1016/J.REHAB.2008.12.011 [retrieved on 2009-02-21]
- KROENKE K ET AL: "Pharmacotherapy of chronic pain: a synthesis of recommendations from systematic reviews", GENERAL HOSPITAL PSYCHIATRY, ELSEVIER SCIENCE PUBLISHING, NEW YORK, NY, US, vol. 31, no. 3, 1 May 2009 (2009-05-01), pages 206-219, XP026085787, ISSN: 0163-8343, DOI: 10.1016/J.GENHOSPPSYCH.2008.12.006 [retrieved on 2009-03-04]
- HO K Y ET AL: "Gabapentin and postoperative pain - a systematic review of randomized controlled trials", PAIN, ELSEVIER SCIENCE PUBLISHERS, AMSTERDAM, NL, vol. 126, no. 1-3, 15 December 2006 (2006-12-15), pages 91-101, XP027886882, ISSN: 0304-3959 [retrieved on 2006-12-15]

## Description

### TECHNICAL FIELD

The invention relates to analgesic pharmaceutical compositions based on sinomenine, an effective component of Chinese herbal medicine *Sinomenium acutum,* and analgesic kits based on such compositions. The present invention also relates to combinations of sinomenine with another analgesic to obtain enhanced analgesic effect during treatments of acute and chronic pain. The compositions can be formulated for clinically accepted slow and/or controlled drug release.

### BACKGROUND

Chronic pain, such as neuropathic pain after injuries to the peripheral and central nervous systems or chronic inflammatory pain resulting from chronic inflammatory diseases such as rheumatoid arthritis, is an important clinical problem. This is because such pain is complex, long-term and resistant to treatments.

The treatment of chronic pain today depends mainly on the use of pharmaceutical drugs, but a considerable portion of patients cannot have effective control of their pain. Currently used analgesics such as opioids have many side effects. The development of tolerance to drugs (a gradual reduction in effect of drugs following long term application) is also a challenge in treating chronic pain. Thus, the three priorities in treatments of chronic pain are (1) increase the efficacy of analgesic effects of the current drugs (2) minimize side effects through decrease in dosage and (3) prevent or delay of occurrence of drug tolerance.

The research on analgesic effects of drugs depends on the use of well-established experimental models. These models include, but not limited to, neuropathic pain models (such as models for spinal cord injury pain or peripheral nerve injury), nerve pathology models from diseases and cancer pain models. Sinomenine (chemical structure shown) is an alkaline substance extracted from the roots of the plant *Sinomenium acutum.* In China, it was used to treat conditions of rheumatism, such as rheumatoid arthritis.

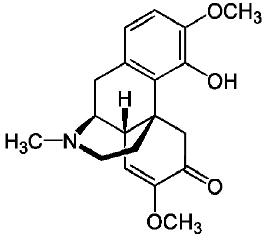

### Chemical structure of sinomenine

In mouse collagen-induced arthritis (CIA) model, Sinomenine treatment can reduce the severity and frequency of arthritis (Huang et al. 2007). Compared with non-steroidal anti-inflammatory drugs (NSAIDS), sinomenine has a better effect on morning stiffness, joint pain and on erythrocyte sedimentation rate (Xu et al. 2008). Sinomenine is also used as an immunosuppressive agent, which inhibits lymphocyte proliferation and the synthesis of B cell antibodies (He et al. 2005). The anti-inflammatory and neuroprotective effects of sinomenine is mediated primarily by decrease of production of superoxide ions through inhibiting the microglial NADPH oxidase (Qian et al. 2007). In addition, sinomenine can also inhibit cyclooxygenase (COX-2), thereby reducing the synthesis of prostaglandin E2 (PGE2) (Liu et al., 1994).

CN1790014 discloses a Chinese medicine composition for treating cancer pain. The composition contains sinomenine, chelidonium and schefflera and is aimed to treat cancer pain, especially cancer of the lung and liver.

CN1214245 discloses new application for sinomenine in making anagesics, especially for the treatment of postoperative pain.

CN101347408 discloses an injectable solution containing Sinomenine to treat chronic pain.

CN101411707 discloses a pharmaceutical composition comprising sinomenine to treat RA and pain caused by RA.

As a drug against RA, the clinical dose range of sinomenine is 200 to 240 mg / day in adults (Xu et al., 2008). There have been no reports about the effect of sinomenine on neuropathic pain.

GABA is an important inhibitory neurotransmitter in the mammalian central nervous system, playing important roles in the function of, for example, cerebral cortex, hippocampus, thalamus, basal ganglia and cerebellum. It modulates many important body functions. A deficiency in GABAergic input will lead to pain, anxiety, restlessness, and fatigue. People under stress, such as athletes or office workers, often require compensation of GABAergic drugs. For example, the clinical analgesic doses for gabapentin in adults are 300 ∼ 600 mg/each time, totaling 900 ∼ 3600 mg / day.

In addition, there have been no reports on synergistic interaction between sinomenine and other drugs in producing analgesia.

### SUMMARY OF THE INVENTION

This invention relates to a pharmaceutical composition for treating chronic pain, especially neuropathic pain, which composition contains sinomenine and other analgesics. We discovered, for the first time, that although sinomenine by itself has no marked analgesic effect, when combined with other analgesics, it can produce significant analgesia through synergism.

Specifically, in the rat model of ischemic spinal cord injury (Xu et al. 1992) and rat or mouse model of peripheral nerve injuries (such as the partial sciatic nerve ischemic injury model) (Kupers et al. 1998, Hao et al. 2000), combination of sinomenine with GABAergic drug such as gabapentin produces significant and synergistic analgesia. In rat and mouse models of acute and chronic inflammation (such as carrageenan-induced inflammation), sinomenine and paracetamol combination produced significant synergism in analgesia. The models used are internationally accepted and representative models of neuropathic pain.

Therefore, this invention provides a pharmaceutical composition for treating pain, wherein the composition contains below indicated (i) and (ii) or consists of (i) and (ii).
(i) sinomenine and/or its acceptable salt, and
(ii)gabapentin or paracetamol.

The composition is particularly applicable for treating neuropathic or acute or chronic inflammatory pain.

This invention also provides a kit for treating pain comprising
(i) a first pharmaceutical composition comprising sinomenine and/or its pharmaceutically acceptable salt, and
(ii)a second pharmaceutical composition comprising gabapentin or paracetamol.
The combination of GABAergic drugs or paracetamol-like drugs with sinomenine, reduces the single dosage requirement for these drugs to 1/4 to 1/10 (or even lower) of the existing clinical doses. It not only enhances the analgesic efficacy of the existing treatments, but also, because of the lower doses, lead to reduction in side effects, preventing and delaying the occurrence of drug tolerance.

### DESCRIPTION OF THE DRAWINGS

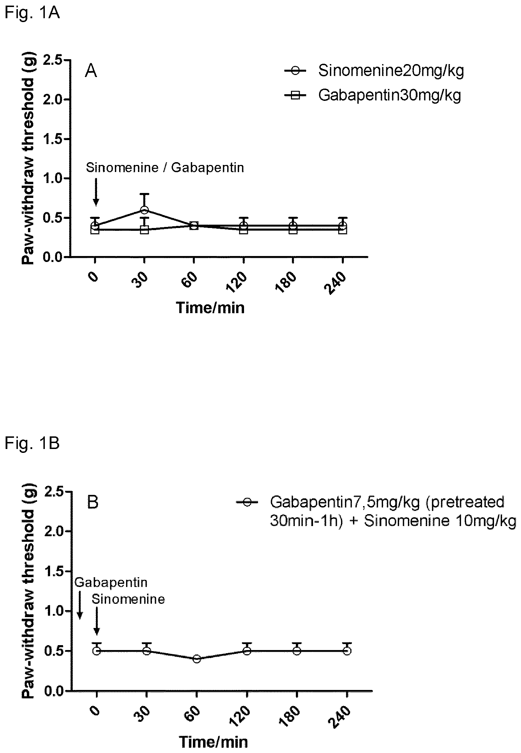

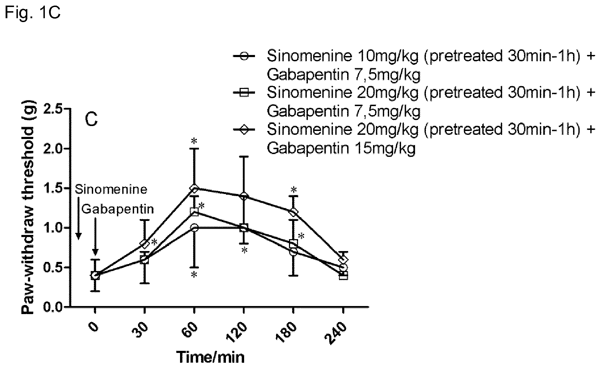

Figure 1A-1C shows the synergistic analgesia by sinomenine and gabapentin in a mouse model of peripheral nerve injury. The analgesic index is hind paw withdrawal threshold to mechanical stimulation (unit gram). Figure 1A shows that sinomenine or gabapentin produced no analgesic effect when administered alone at given doses; Figure 1B shows that gabapentin produced no analgesic effect when administered prior to sinomenine. However, Figure 1C shows small doses of drug combination produced significant analgesic effect when sinomenine was given 30-60 min prior to gabapentin.

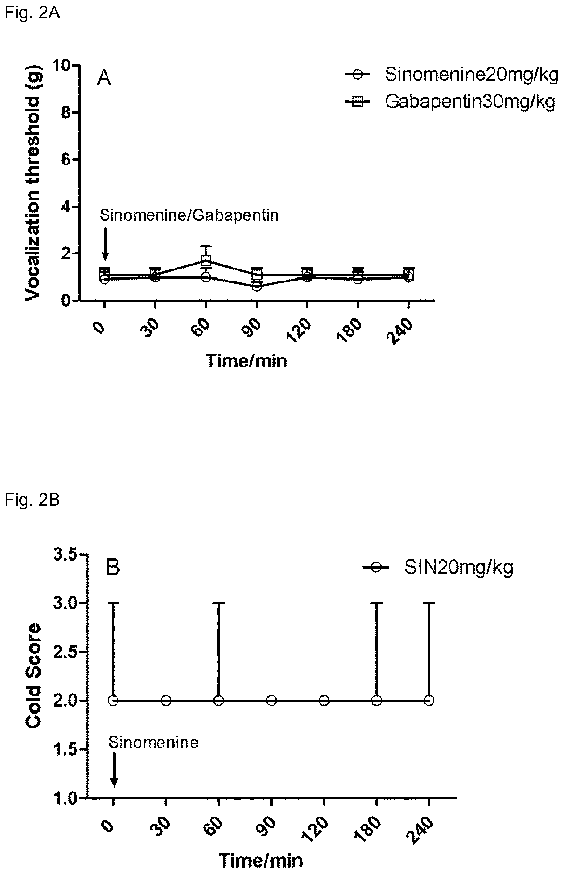

Figure 2A-2D show the synergistic analgesic effect of sinomenine and gabapentin in the rat model of spinal cord injury. The analgesic index is response threshold to mechanical stimulation (grams) or response scores to cold stimulation. Figures 2A and 2B show that sinomenine and gabapentin, when administered alone, produced no analgesia to mechanical or cold stimulation;

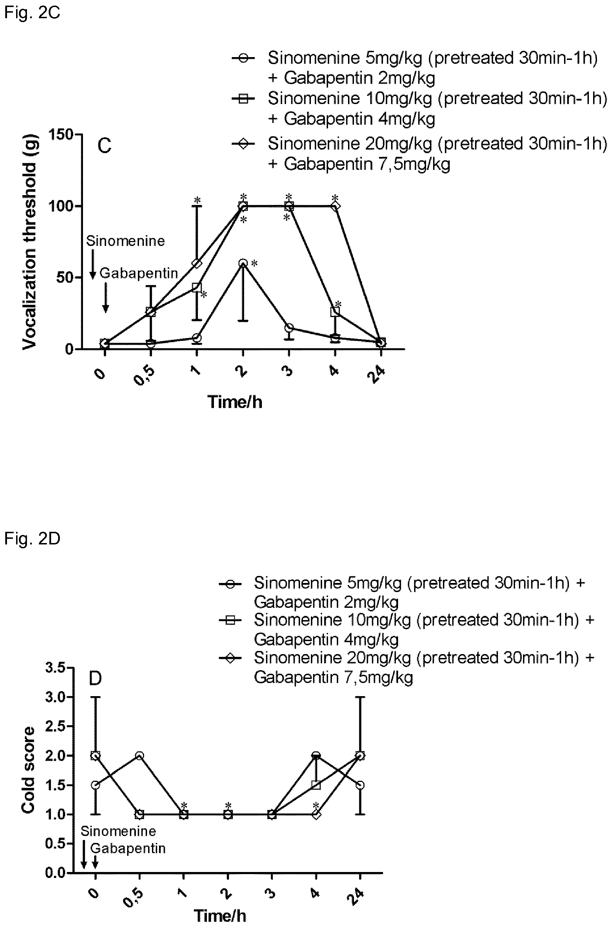

Figures 2C and 2D show small doses of drug combination produced significant analgesic effect when sinomenine was given 30-60 min prior to gabapentin against both mechanical and cold stimulation.

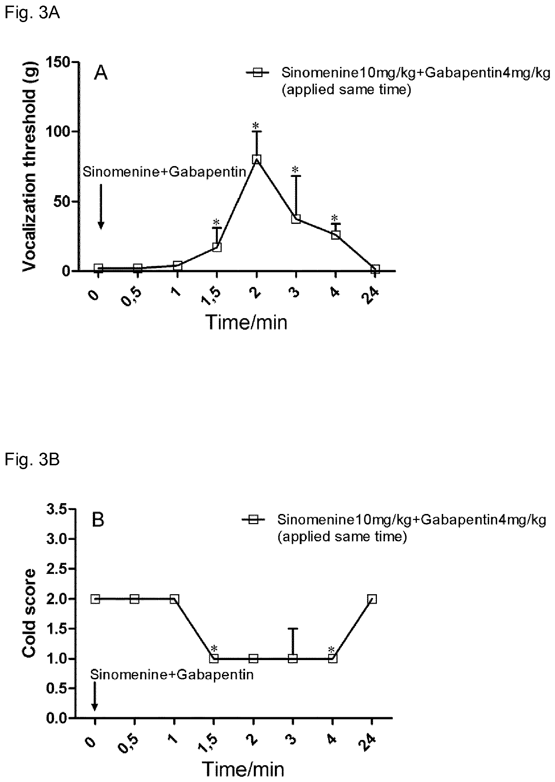

Figure 3A and 3B show the synergistic analgesic effect of sinomenine and gabapentin given simultaneously in a rat model of spinal cord injury. The analgesic index is response threshold to mechanical stimulation (grams) or response scores to cold stimulation. Simultaneous administration of 10 mg / kg sinomenine and 4 mg / kg gabapentin produce significant analgesic effect.

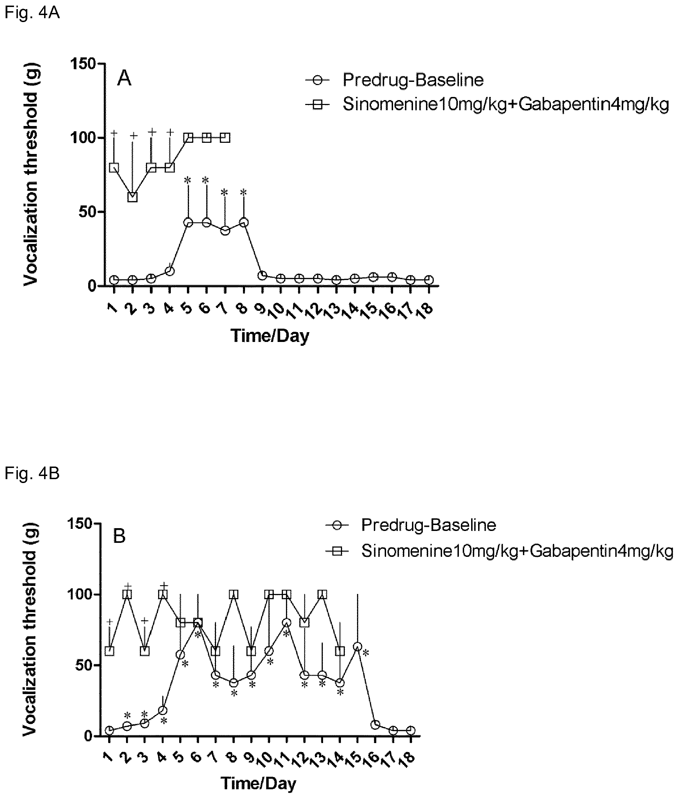

Figure 4A and 4B show the synergistic analgesic effect of chronic administration of small doses of sinomenine and gabapentin in the rat model of spinal cord injury. The analgesic index is response threshold to mechanical stimulation (grams). Co-administration of low doses of combined sinomenine and gabapentin daily for 7 or 14 days produced sustained analgesic effect. No tolerance to the effect was observed.

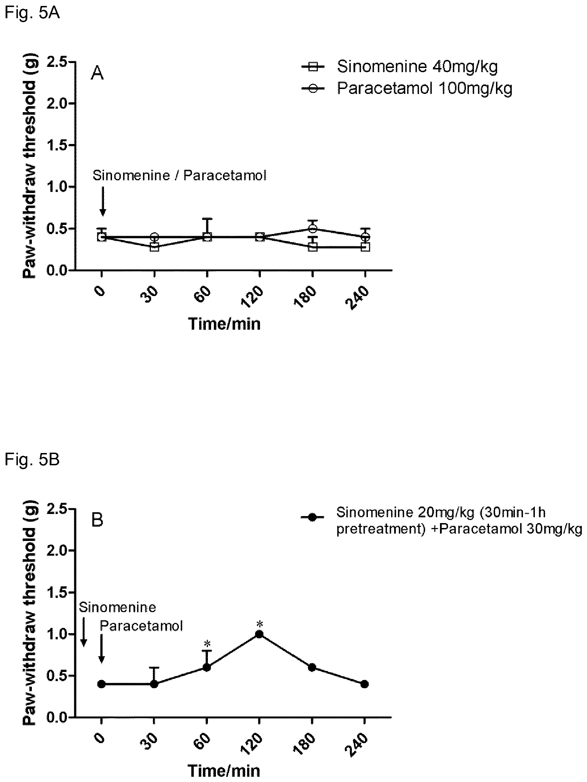

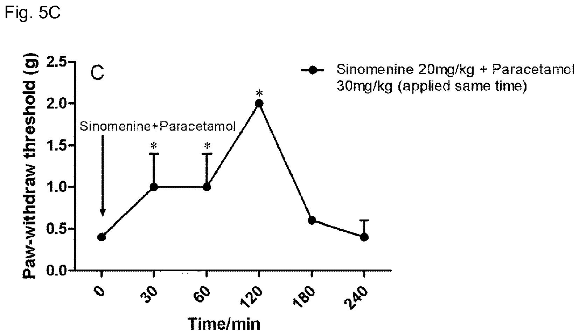

Figure 5A-5C show the synergistic analgesic effect co-administration of sinomenine and paracetamol in carrageenan-induced inflammatory pain in mice. The pain index is hind paw withdrawal threshold to mechanical stimulation (grams). Figure 5A shows sinomenine or paracetamol, when administered alone at given doses, does not produce analgesia; Figure 5B and C shows that combination of low doses of sinomenine and gabapentin produces significant analgesia when sinomenine is administered 30 minutes to 1 hour before gabapentin or at the same time as gabapentin.

### DETAILED DESCRIPTION OF THE INVENTION

The present invention provides a pharmaceutical composition for treatment of pain. The composition contains below indicated (i) and (ii) or consists of (i) and (ii).
(i) sinomenine and/or its acceptable salt, and
(ii)gabapentin or paracetamol.

Wherein said (i) and (ii) may be present in separate form, or in the form of a pharmaceutical mixture.

In the products of composition where (i) and (ii) are present in separate forms, the practical application is that (i) and (ii) will be packaged separately in single doses of respective formulations. In particular, the form of separate packaging will enable the release of (i) sinomenine and /or its acceptable salt thereof prior to the release of (ii) GABAergic drugs or paracetamol-like drugs. Alternatively, such packaging can also enable the simultaneous release of (i) and (ii). In such condition, the preferable dose range for the GABAergic drug gabapentin will be 50 to 200 mg, paracetamol 50 to 400 mg and sinomenine 50 mg to 200 mg. The quantity ratio for single dose formulation will be in the range of 1:1 to 3:1 for sinomenine/gabapentin (e.g. 1.3:1 or 2.5:1) and in the range of 1:1 to 1:2 for sinomenine/paracetamol, (e.g. 1:1.5). Such dose ratio may be adjusted based on results from clinical trials.

In the products of composition where (i) and (ii) are present in a mixed form, what is described is a pharmaceutical mixture. This pharmaceutical mixture should consist of the (i), (ii) and other pharmaceutically applicable excipients, diluents and/or carriers. In such pharmaceutical composition, In such condition, the preferable dose range for the GABAergic drug gabapentin will be 50 to 200 mg, paracetamol 50 to 400 mg and sinomenine 50 mg to 200 mg. The quantity ratio for single dose formulation will be in the range of 1:1 to 3:1 for sinomenine/gabapentin (e.g. 1.3:1 or 2.5:1) and in the range of 1:1 to 1:2 for sinomenine/paracetamol, (e.g. 1:1.5). Such dose ratio may be adjusted based on results from clinical trials.

The clinical pain indication relevant for the pharmaceutical compositions and products from the present invention includes: (a) acute pain such as postoperative pain and severe visceral pain; (b) chronic pain such as cancer pain, arthritis pain, and back pain; and (c) neuropathic pain, such as nerve and spinal cord injury pain, post-stroke pain, diabetic neuropathy induced pain, pain caused by the herpes virus, and trigeminal neuralgia and other types of facial pain.

This invention, through sinomenine based drug combinations, achieves (1) enhancement of analgesic effects of existing drugs, and (2) decrease of side effects of drugs through reduction in doses of drugs used, and (3) prevention and delay the occurrence of drug tolerance. This invention has broad prospects in application. Accordingly, the present invention further involves the practical aspects of producing the pharmaceutical composition of sinomenine or its acceptable salt thereof with GABAergic drugs or paracetamol-like drugs for pain relief.

The pharmaceutical composition can be used for the treatment, amelioration and mitigation of pain after nerve injury. In one embodiment, the pharmaceutical composition consists of sinomenine and gabapentin in separate forms. For application, sinomenine was administered first, 30-60 minutes prior to gabapentin and the preferable doses for sinomenine is 10-20 mg/kg in mice (or equivalent human doses) or 5-20 mg/kg in rats (or equivalent human doses). The preferable doses for gabapentin is 7.5 mg-15 mg/ kg in mice (or equivalent human doses) or 2-7.5 mg/kg in rats (or equivalent human doses). In this and following examples, the human doses can be calculated based on dosing data in rodents disclosed in this invention and human body weight using known methods (e.g., the methods of weight or body surface area) with also consideration to known clinical doses for these drugs. The pharmaceutical composition can be formulated in anyone of the dosage forms adapted for effective dosing regimen of the composition and for any sequence of administration to subjects (e.g., mouse, rat, human) with pain after nerve injury.

In another embodiment for the treatment, amelioration and mitigation of pain after nerve injury the pharmaceutical composition consists of sinomenine and gabapentin in a mixed form. The pharmaceutical mixture should have sinomenine at the dose of 10 mg/kg in rats (or equivalent human doses) and gabapentin at 4 mg/kg in rats (or equivalent human doses). The pharmaceutical composition can be formulated in anyone of the dosage forms adapted for effective dosing regimen of the composition for subjects (e.g., mouse, rat, human) with pain after nerve injury.

In yet another embodiment for the treatment, amelioration and mitigation of pain after nerve injury the pharmaceutical composition consists of sinomenine and gabapentin in separate forms. For application, the composition was administered twice a day for 7 or 14 days, each administration contains sinomenine at 10 mg/kg in rats (or equivalent human doses) followed by gabapentin 30-60 min later at 4 mg/ kg in rats (or equivalent human doses). The pharmaceutical composition can be formulated in anyone of the dosage forms adapted for effective dosing regimen of the composition and for any sequence, duration and frequency of administration to subjects (e.g., mouse, rat, human) with pain after nerve injury.

The pharmaceutical composition can also be used for the treatment, amelioration and mitigation of pain associated with inflammation. In one embodiment, the pharmaceutical composition consists of sinomenine and paracetamol in separate forms. For application, sinomenine was administered first, 30-60 minutes prior to paracetamol and the preferable dose for sinomenine is 20 mg/kg in mice (or equivalent human doses) and the preferable dose for paracetamol is 30 mg/ kg in mice (or equivalent human doses). The pharmaceutical composition can be formulated in anyone of the dosage forms adapted for effective dosing regimen of the composition and for any sequence of administration to subjects (e.g., mouse, rat, human) with pain after inflammation.

In yet another embodiment for the treatment, amelioration and mitigation of pain associated with inflammation the pharmaceutical composition consists of sinomenine and paracetamol in a mixed form. The pharmaceutical mixture should have sinomenine at the dose of 20 mg/kg in mice (or equivalent human doses) and paracetamol at 30 mg/kg in mice (or equivalent human doses). The pharmaceutical composition can be formulated in anyone of the dosage forms adapted for effective dosing regimen of the composition for subjects (e.g., mouse, rat, human) with pain after inflammation.

In all the listed applicable embodiments of this invention, the single doses of gabapentin used for synergistic interaction with sinomenine are 7.5-15 mg/kg mice weight or 2-7.5 mg/kg rats weight. This is equivalent to human doses of 20-75 mg (estimated according to body surface area method, considering adult body weight of 60 kg) and much below the regular single oral dose for adult human for analgesia which is 300-600 mg. For chronic administration, the dose of gabapentin used is 4 mg/kg rats weight per injection and 8 mg/kg rat weight per day. This is equivalent to 80mg/day in humans which is far lower than the regular human oral doses for chronic gabapentin which is 900-3600 mg per day. In addition, the single doses of paracetamol used for synergistic interaction with sinomenine are 30 mg/kg mice weight. This is equivalent to human doses of 150 mg which is significantly lower than the regular single oral dose for adult human for analgesia which is 500-1000 mg. Thus, this invention of pharmaceutical compositions, products and kits can be used for the treatment, amelioration and mitigation of pain using much lower doses of conventional analgesics to achieve analgesia.

Further, the human equivalent dose of sinomenine is about 200 mg / day (chronic administration) or 50 ∼ 200 mg as single administration. These doses do not exceed adult human doses of sinomenine that have been in use. Because gabapentin and paracetamol are existing analgesics, when applied to the present invention of pharmaceutical compositions or products, the doses of gabapentin and paracetamol may be higher than the above discussed human equivalent doses as long as they do not exceed accepted adult doses for respective drugs. Further, the human doses may also vary based on the actual weight of the patient, physical conditions and the results of other clinical trials and clinical doses in use. Therefore, the actual administered doses in the composition may fluctuate, for example, for adult patients, the single dose of gabapentin may be 50 ∼ 200 mg and single dose of paracetamol may 50 ∼ 400 mg.

For professionals in the medical field, it should be understood that the analgesics used for synergism with sinomenine are not limited to the examples. Analgesics that belong to the same type of drugs as the examples are also useful. For example, GABAergic drugs can be gabapentin and pregabalin. Paracetamol-like drugs can be paracetamol, aspirin and ibuprofen.

The time differences for drug application in the pharmaceutical compositions listed in this invention can be achieved by administration of drugs at different times. However, for professionals in the medical field, such time differences can also be achieved by clinically acceptable drug delivery technology. For example, within a single administration of the composition, gabapentin may be packaged into capsules with slow release property. Sinomenine and gabapentin can also be put into different capsules with different release characteristics to achieve the desired time differences.

Unless otherwise noted, when used herein this document, the unit of a pharmaceutical dosage "mg / kg" refers to the subject's body weight per kg corresponding to doses of drugs in mg.

### EXAMPLES

The following examples will further illustrate the embodiments within the scope of the present invention. However, the examples are given solely for the purpose of illustration and are not to be construed as limitations of the present invention. Unless otherwise specified, the route of administration of all drugs are the same in the examples.

### [EXAMPLE 1]

Synergistic analgesic effect of gabapentin and sinomenine in a mouse model of peripheral neuropathic pain

In a mouse partial sciatic nerve injury model, we can observe mechanical hyperalgesia, manifested as decrease in response threshold to mechanical stimulation. Single oral administration of sinomenine 20 mg/kg has no effect on the response threshold observed up to 4 hours (tested every 30-60 min) after administration (Figure 1A). Single oral administration of gabapentin 30 mg/kg also has no effect on the response threshold observed up to 4 hours (tested every 30-60 min) after administration (Figure 1A). When used in low dose combination, the first administration of 7.5 mg/kg of gabapentin was followed 30 minutes to 1 hour later by administration of 10 mg/kg sinomenine. No effect was observed on response threshold up to 4 hours after administration (tested every 30-60 min) (Figure 1 B). However, when the first application of sinomenine followed 30-60 min later by gabapentin, significant analgesia can be observed up to 4 hours observation (tested every 30-60 min) (see Figure 1 C). The analgesic effect increased following increase in doses of the composition. The three dose combination tested are "sinomenine + gabapentin," at "10 mg/kg +7.5 mg/kg" "20 mg/kg +7.5 mg/kg" and "20 mg/kg +15 mg/kg. All combinations produced no side effects in mice. The results are the average of the threshold of 6 mice in each group.

### [EXAMPLE 2]

### Synergistic analgesic effect of sinomenine and gabapentin in rats with spinal cord injury

In the rat model of spinal cord injury, we can also observe a strong hypersensitivity to mechanical and cold stimulation. When administered alone (intraperitoneal injection) 20 mg/kg of sinomenine or 30 mg/kg gabapentin did not produce analgesia against mechanical stimulation (response threshold in grams) or cold stimulation studied with a scoring system to a cold spray (Figure 2A and 2B). However, when administered in combination, small doses of sinomenine and gabapentin (sinomenine administered 30-60 min before gabapentin) produced a strong analgesic effect observed up to 24 hours (see Figure 2C and 2D). The three dose combinations are "sinomenine + gabapentin," at "5 mg/kg +2 mg/kg" "10 mg/kg +4 mg/kg" and "20 mg/kg +7.5 mg/ kg".

In addition, simultaneous administration of 10 mg/kg sinomenine and 4 mg/kg gabapentin can be produce a significant synergistic analgesia (Figure 3A and 3B).

The above combinations produced no observable adverse effects in rats. Results were average of 6-11 rats in each group.

### [EXAMPLE 3]

### Sinomenine and gabapentin produced synergistic analgesia in chronic administration

In the rat model of spinal cord injury pain, we select the 4 mg/kg of gabapentin and 10 mg/kg of sinomenine in combination to study the effects of chronic administration. In two rounds of experiments, twice daily administration (intraperitoneal injection) of sinomenine followed by gabapentin 30-60 min later were performed with dosing interval of 6 hours for each day. The results are shown in Figure 4A and 4B, the combined administration for 7 days (Fig. 4A) or 14 days (Figure 4B) of sinomenine and gabapentin produce significant analgesic effect and no tolerance development. More importantly, in the course of chronic administration, the pain threshold before the administration was also significantly increased, which indicates that the drug combination produced a sustained analgesia. In about 2 days after termination of last drug administration, the pain threshold of rats returned to pre-treatment level. The drug composition in rats did not produce observable side effects during chronic administration. Results are the average threshold of six rats in each group.

### [EXAMPLE 4]

The synergistic effect of co-administration of sinomenine and paracetamol in mice after carrageenan-induced inflammation

In mouse carrageenan-induced inflammatory model, we can observe mechanical hyperalgesia. Single oral administration sinomenine at 40 mg/kg or paracetamol alone at 100mg/kg had no significant analgesic effect up to 4 hours after administration (tested every 30-60 min) (Figure 5A). When combined at small doses, sinomenine at 20 mg/kg administered 30-60 min prior to 30 mg/kg paracetamol produced significant analgesia up to 4 hours after administration (Figure 5B). In addition, simultaneous administration of 20 mg/kg of sinomenine and 30 mg/kg paracetamol, can also produces a significant synergistic effect (Figure 5C). The above combination in mice produced no observable adverse effects. Results are the average threshold of 5-6 mice in each group.

From these examples, it is suggested that one important application of the invention is to achieve an effective analgesic effect by a composition of small doses of the drugs, leading to reduction of side effects and drug tolerance. It has been clearly demonstrated that when used alone, all three tested compounds, sinomenine, gabapentin and paracetamol cannot produce desired effect at rather high doses. However, a combination of small doses of these compouns, especially applied in the right order, can produce effective analgesia with no side effects.

The present invention covers combination of sinomenine with two types of clinical used analgesics, gabapentin and paracetamol. The present invention will lead to the development of one or more new drugs with compositions of different components and doses. These drug may provide patients worldwide with effective pain relief, reduced side effects and drug tolerance. The range of disease conditions include, but are not limited to, inflammation, arthritis, cancer, diabetes, herpes, nerve damage, AIDS, complications of chemotherapy, stroke and spinal cord injury.

### REFERENCES

Hao JX, Blakeman KH, Yu W, Hultenby K, Xu XJ, Wiesenfeld-Hallin Z. Development of a mouse model of neuropathic pain following photochemically induced ischemia in the sciatic nerve Exp Neurol 2000;. 163:231-8.
He XB, Wang JL, XH Guo, Liu QY, Chen TY, Cao XT, et al .. Requirement for ERK activation in sinomenine-induced apoptosis of macrophages Immunology Letters 2005; 98:. 91-96.
Huang F, Yamaki K, Takano H, Inoue K, Yanagisawa R, Yoshino S. Effect of sinomenine on collagen-induced arthritis in mice Autoimmunity 2007; 40 (7):. 532-539.
Kupers R, Yu W, Persson JKE, Xu XJ, Wiesenfeld-Hallin Z. Photochemically-induced ischemia of the rat sciatic nerve produces a dose-dependent and highly reproducible mechanical, heat and cold allodynia, and signs of spontaneous pain Pain 1998.; 76:45-59.
Liu L, Riese J, Resch K, Kaever V. Impairment of macrophage eivosanoid and nitric oxide production by an alkaloid from Sinomenum acutum Arzneimittelforschung 1994; 44:. 1123-6.
Qian L, Xu ZL, Zhang W, Wilson B, Hong JS and Flood MP Sinomenine, a natural dextrorotatory morphinan analog, is anti-inflammatory and neuroprotective through inhibition of microglial NADPH oxidase Journal of Neuroinflammation 2007;. 4 (23) Xu. M, Liu L, Qi C, Deng B, Cai X. Sinomenine Versus NSAIDs for the Treatment of Rheumatoid Arthritis: A Systemic Review and Meta-Analysis Bibliography 2008; 74:. 1423-9.
Xu X.-J "Hao J.-X., Aldskogius H., Seiger A., Wiesenfeld-Hallin Z. Chronic pain-related syndrome in rats after ischemic spinal cord lesion:. A possible animal model for pain in patients with spinal . cord injury Pain 1992; 48: 279-90.

## Claims

1. A pharmaceutical composition for treating pain comprising:
(i) sinomenine and/or its pharmaceutically acceptable salt; and
(ii) gabapentin or paracetamol.

2. A pharmaceutical composition according to claim 1, wherein said (i) and (ii) are present in mutually isolated forms, or in the form of a mixture of said (i) and (ii).

3. A pharmaceutical composition according to claim 2, wherein the isolated forms are single-dose formulations of each of said (i) and (ii) which are packaged separately.

4. A pharmaceutical composition according to claim 2, wherein the isolated forms comprise formulations enabling release of said (i) being sinomenine and/or its pharmaceutically acceptable salt prior to release of said (ii) being gabapentin or paracetamol; or formulations enabling simultaneous release of said (i) and (ii) from a same single-dose formulation comprising said (i) and (ii).

5. A pharmaceutical composition according to claim 3 or 4, wherein the single-dose formulation comprises sinomenine in a range of 50 mg - 200 mg and gabapentin in a range of 50 mg - 200 mg, or sinomenine in a range of 50 mg - 200 mg and paracetamol in a range of 50 mg - 400 mg.

6. A pharmaceutical composition according to claim 3 or 4, wherein the weight ratio of said (i) being sinomenine and/or its pharmaceutically acceptable salt and said (ii) being gabapentin in the single-dose formulation is 3:1 to 1:1; the weight ratio of said (i) being sinomenine and/or its pharmaceutically acceptable salt and said (ii) being paracetamol in the single-dose formulation is 1:1 to 1:2.

7. A pharmaceutical composition according to claim 2 further comprising pharmaceutically acceptable excipients wherein said (i) and (ii) are present in the form of a mixture of said (i) and (ii), and wherein the weight ratio of said (i) being sinomenine and/or its pharmaceutically acceptable salt and said (ii) being gabapentin is 3:1 to 1:1; the weight ratio of said (i) being sinomenine and/or its pharmaceutically acceptable salt and said (ii) being paracetamol is 1:1 to 1:2.

8. A pharmaceutical composition according to claim 7, wherein a single-dose formulation of the pharmaceutical composition comprises sinomenine in a range of 50 mg - 200 mg and gabapentin in a range of 50 mg - 200 mg; or sinomenine in a range of 50 mg - 200 mg and paracetamol- in a range of 50 mg - 400 mg.

9. A pharmaceutical composition according to claims 1 to 8 for use in the treatment of pain caused by neuropathology, disease-resulted peripheral nerve disorders and/or cancer.

10. A pharmaceutical composition according to claims 1 to 8 for use in the treatment of neuropathic pain, acute and/or chronic inflammatory pain, including pain caused by spinal cord injury, lesion in the peripheral nervous system and/or inflammation.

11. A kit for treating pain comprising
(i) a first pharmaceutical composition comprising sinomenine and/or its pharmaceutically acceptable salt, and
(ii) a second pharmaceutical composition comprising gabapentin or paracetamol.

12. A kit according to claims 11 for use in the treatment of pain caused by neuropathology, disease-resulted peripheral nerve disorders and/or cancer.

13. A kit according to claims 11 for use in the treatment of neuropathic pain, acute and/or chronic inflammatory pain, including pain caused by spinal cord injury, lesion in the peripheral nervous system and/or inflammation.

## Patentansprüche

1. Pharmazeutische Zusammensetzung zum Behandeln von Schmerz, umfassend:
(i) Sinomenin und/oder sein pharmazeutisch verträgliches Salz; und
(ii) Gabapentin oder Paracetamol.

2. Pharmazeutische Zusammensetzung gemäß Anspruch 1, wobei die (i) und (ii) in voneinander isolierten Formen oder in der Form eines Gemischs der (i) und (ii) vorliegen.

3. Pharmazeutische Zusammensetzung gemäß Anspruch 2, wobei die isolierten Formen Einzeldosis-Formulierungen von jedem der (i) und (ii) sind, die getrennt verpackt sind.

4. Pharmazeutische Zusammensetzung gemäß Anspruch 2, wobei die isolierten Formen Formulierungen umfassen, die die Freisetzung des (i), das Sinomenin und/oder sein pharmazeutisch verträgliches Salz ist, vor der Freisetzung des (ii), das Gabapentin oder Paracetamol ist, ermöglichen; oder Formulierungen, die gleichzeitige Freisetzung der (i) und (ii) aus einer gemeinsamen Einzeldosisformulierung, die die (i) und (ii) umfasst, ermöglichen.

5. Pharmazeutische Zusammensetzung gemäß Anspruch 3 oder 4, wobei die Einzeldosisformulierung Sinomenin in einem Bereich von 50 mg bis 200 mg und Gabapentin in einem Bereich von 50 mg bis 200 mg oder Sinomenin in einem Bereich von 50 mg bis 200 mg und Paracetamol in einem Bereich von 50 mg bis 400 mg umfasst.

6. Pharmazeutische Zusammensetzung gemäß Anspruch 3 oder 4, wobei das Gewichtsverhältnis des (i), das Sinomenin und/oder sein pharmazeutisch verträgliches Salz ist, und des (ii), das Gabapentin ist, in der Einzeldosisformulierung 3:1 bis 1:1 beträgt; das Gewichtsverhältnis des (i), das Sinomenin und/oder sein pharmazeutisch verträgliches Salz ist, und des (ii), das Paracetamol ist, in der Einzeldosisformulierung 1:1 bis 1:2 beträgt.

7. Pharmazeutische Zusammensetzung gemäß Anspruch 2, ferner umfassend pharmazeutisch verträgliche Hilfsstoffe, wobei die (i) und (ii) in der Form eines Gemischs der (i) und (ii) vorliegen und wobei das Gewichtsverhältnis des (i), das Sinomenin und/oder sein pharmazeutisch verträgliches Salz ist, und des (ii), das Gabapentin ist, 3:1 bis 1:1 beträgt; das Gewichtsverhältnis des (i), das Sinomenin und/oder sein pharmazeutisch verträgliches Salz ist, und des (ii), das Paracetamol ist, 1:1 bis 1:2 beträgt.

8. Pharmazeutische Zusammensetzung gemäß Anspruch 7, wobei eine Einzeldosisformulierung der pharmazeutischen Zusammensetzung Sinomenin in einem Bereich von 50 mg bis 200 mg und Gabapentin in einem Bereich von 50 mg bis 200 mg; oder Sinomenin in einem Bereich von 50 mg bis 200 mg und Paracetamol in einem Bereich von 50 mg bis 400 mg umfasst.

9. Pharmazeutische Zusammensetzung gemäß Ansprüchen 1 bis 8 für die Verwendung bei der Behandlung von Schmerz, der von Neuropathologie, krankheitsbedingten peripheren Nervenstörungen und/oder Krebs verursacht ist.

10. Pharmazeutische Zusammensetzung gemäß Ansprüchen 1 bis 8 für die Verwendung bei der Behandlung von neuropathischem Schmerz, akutem und/oder chronischem Entzündungsschmerz, einschließlich Schmerz, der von Rückenmarkverletzung, Läsion in dem peripheren Nervensystem und/oder Entzündung verursacht ist.

11. Kit zum Behandeln von Schmerz, umfassend
(i) eine erste pharmazeutische Zusammensetzung, umfassend Sinomenin und/oder sein pharmazeutisch verträgliches Salz und
(ii) eine zweite pharmazeutische Zusammensetzung, umfassend Gabapentin oder Paracetamol.

12. Kit gemäß Anspruch 11 für die Verwendung bei der Behandlung von Schmerz, der von Neuropathologie, krankheitsbedingten peripheren Nervenstörungen und/oder Krebs verursacht ist.

13. Kit gemäß Anspruch 11 für die Verwendung bei der Behandlung von neuropathischem Schmerz, akutem und/oder chronischem Entzündungsschmerz, einschließlich Schmerz, der von Rückenmarkverletzung, Läsion in dem peripheren Nervensystem und/oder Entzündung verursacht ist.

## Revendications

1. Composition pharmaceutique pour le traitement de la douleur comprenant :
(i) de la sinoménine et/ou son sel pharmaceutiquement acceptable ; et
(ii) de la gabapentine ou du paracétamol.

2. Composition pharmaceutique selon la revendication 1, où lesdits (i) et (ii) sont présents sous des formes mutuellement isolées, ou sous la forme d'un mélange desdits (i) et (ii).

3. Composition pharmaceutique selon la revendication 2, où les formes isolées sont des formules de dose unitaire de chacun desdits (i) et (ii) qui sont conditionnés séparément.

4. Composition pharmaceutique selon la revendication 2, où les formes isolées comprennent des formules permettant la libération dudit (i) qui est la sinoménine et/ou son sel pharmaceutiquement acceptable avant la libération dudit (ii) qui est la gabapentine ou le paracétamol ; ou des formules permettant la libération simultanée desdits (i) et (ii) à partir d'une même formule de dose unitaire comprenant (i) et (ii).

5. Composition pharmaceutique selon la revendication 3 ou 4, où la formule de dose unitaire comprend de la sinoménine dans un intervalle de 50 mg à 200 mg et de la gabapentine dans un intervalle de 50 mg à 200 mg, ou de la sinoménine dans un intervalle de 50 mg à 200 mg et du paracétamol dans un intervalle de 50 mg à 400 mg.

6. Composition pharmaceutique selon la revendication 3 ou 4, où le rapport massique dudit (i) qui est la sinoménine et/ou son sel pharmaceutiquement acceptable sur ledit (ii) étant la gabapentine dans la formule de dose unitaire est compris entre 3:1 et 1:1 ; le rapport massique dudit (i) qui est la sinoménine et/ou son sel pharmaceutiquement acceptable sur ledit (ii) qui est le paracétamol dans la formule de dose unitaire est compris entre 1:1 et 1:2.

7. Composition pharmaceutique selon la revendication 2 comprenant en outre des excipients pharmaceutiquement acceptables où lesdits (i) et (ii) sont présents sous la forme d'un mélange desdits (i) et (ii), et où le rapport massique dudit (i) qui est la sinoménine et/ou son sel pharmaceutiquement acceptable sur ledit (ii) qui est la gabapentine est compris entre 3:1 et 1:1, le rapport massique dudit (i) et la sinoménine et/ou son sel pharmaceutiquement acceptable sur ledit (ii) qui est le paracétamol est compris entre 1:1 et 1:2.

8. Composition pharmaceutique selon la revendication 7, où la formule de dose unitaire de la composition pharmaceutique comprend de la sinoménine dans un intervalle de 50 mg à 200 mg et de la gabapentine dans un intervalle de 50 mg à 200 mg ; ou de la sinoménine dans un intervalle de 50 mg à 200 mg et du paracétamol dans un intervalle de 50 mg à 400 mg.

9. Composition pharmaceutique selon les revendications 1 à 8 pour utilisation dans le traitement de la douleur provoquée par une pathologie neuronale, des troubles des nerfs périphériques résultant d'une maladie et/ou le cancer.

10. Composition pharmaceutique selon les revendications 1 à 8 pour utilisation dans le traitement de la douleur névropathique, de la douleur inflammatoire aiguë et/ou chronique, y compris la douleur provoquée par les lésions de la moelle épinière, les lésions du système nerveux périphérique et/ou l'inflammation.

11. Kit de traitement de la douleur comprenant
(i) une première composition pharmaceutique comprenant de la sinoménine et/ou son sel pharmaceutiquement acceptable, et
(ii) une deuxième composition pharmaceutique comprenant de la gabapentine ou du paracétamol.

12. Kit selon la revendication 11 pour utilisation dans le traitement de la douleur provoquée par une pathologie neuronale, des troubles des nerfs périphériques résultant d'une maladie et/ou le cancer.

13. Kit selon la revendication 11 pour utilisation dans le traitement de la douleur névropathique, de la douleur inflammatoire aiguë et/ou chronique, y compris la douleur provoquée par les lésions de la moelle épinière, les lésions du système nerveux périphérique et/ou l'inflammation.
